(19) Europäisches Patentamt
European Patent Office
Office européen des brevets

(11) Veröffentlichungsnummer: **0 116 854**
**B1**

(12) # EUROPÄISCHE PATENTSCHRIFT

(45) Veröffentlichungstag der Patentschrift: **12.10.88**

(51) Int. Cl.⁴: **C 07 D 405/04**, C 07 D 417/12

(21) Anmeldenummer: **84100452.6**

(22) Anmeldetag: **17.01.84**

(54) **Verfahren zur Herstellung von optisch einheitlichen 2-Azetidinon-Derivaten.**

(30) Priorität: **20.01.83 CH 320/83**

(43) Veröffentlichungstag der Anmeldung:
**29.08.84 Patentblatt 84/35**

(45) Bekanntmachung des Hinweises auf die Patenterteilung:
**12.10.88 Patentblatt 88/41**

(84) Benannte Vertragsstaaten:
**AT BE CH DE FR GB IT LI LU NL SE**

(56) Entgegenhaltungen:
**EP-A-0 073 061**
**EP-A-0 096 296**
**EP-A-0 101 598**

**CHEMICAL ABSTRACTS, Band 94, Nr. 17, 27. April 1981, Seite 736, Nr. 139522f, Columbus, Ohio, US; S.D. SHARMA et al.: "Some novel monocyclic cis-beta-lactams from glycine" & INDIAN J. CHEM., SECT. B 1980, 19B(9), 760-4**

(73) Patentinhaber: **F. HOFFMANN- LA ROCHE & CO. Aktiengesellschaft, CH- 4002 Basel (CH)**

(72) Erfinder: **Schmid, Gérard, Dr., Mittler Feldweg 6, CH- 4468 Kienberg (CH)**

(74) Vertreter: **Lederer, Franz, Dr., Van der Werth, Lederer & Riederer Patentanwälte Lucile- Grahn-Strasse 22, D-8000 München 80 (DE)**

**EP 0 116 854 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein Verfahren zur Herstellung von neuen, optisch einheitlichen β-Lactamen der allgemeinen Formel

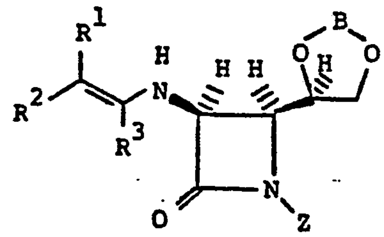

I

worin $R^1$ niederes Alkanoyl, niederes Alkoxycarbonyl oder Benzoyl, $R^2$ Wasserstoff, niederes Alkyl, niederes Alkanoyl, niederes Alkoxycarbonyl oder Cyano, $R^3$ Wasserstoff oder niederes Alkyl, B niederes Alkyliden, niederes Cycloalkyliden oder Carbonyl und Z niederes 2-Alkenyl oder 2,4-Di(niederes Alkoxy)benzyl bedeuten,

und ihren entsprechenden optischen Antipoden, welches dadurch gekennzeichnet ist, dass man ein Salz einer Carbonsäure der allgemeinen Formel

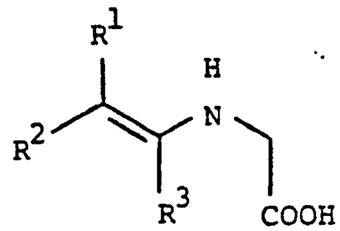

II

worin $R^1$, $R^2$ und $R^3$ obige Bedeutung besitzen, in Gegenwart eines reaktiven Derivates einer organischen Sulfonsäure und einer Base mit einer optisch einheitlichen Verbindung der allgemeinen Formel

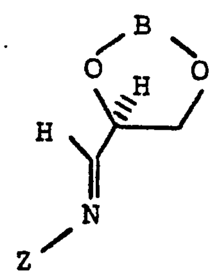

III

worin B und Z obige Bedeutung besitzen, oder dem optischen Antipoden davon umsetzt.

Der Ausdruck "nieder" bezeichnet Reste und Verbindungen mit höchstens 7, vorzugsweise höchstens 4 Kohlenstoffatomen. Der Ausdruck "Alkyl" bezeichnet geradkettige oder verzweigte, gesättigte Kohlenwasserstoffreste, wie Methyl, Äthyl und Isopropyl. Der Ausdruck "Alkoxy" bezeichnet Alkyläthergruppen, wie Methoxy und Äthoxy. Der Ausdruck "Alkanoyl" bezeichnet geradkettige oder verzweigte, gesättigte Fettsäurereste, wie Formyl und Acetyl. Der Ausdruck "2-Alkenyl" bezeichnet geradkettige oder verzweigte Kohlenwasserstoffreste mit mindestens 3 Kohlenstoffatomen, welche in der 2,3-Stellung eine Doppelbindung aufweisen, wie Allyl. Der Ausdruck "Alkyliden" bezeichnet Reste, wie Methylen und Isopropyliden. Der Ausdruck "Cycloalkyliden" bezeichnet cyclische Kohlenwasserstoffreste mit mindestens 3 Kohlenstoffatomen, wie Cyclohexyliden.

Der weiter unten verwendete Ausdruck "Aryl" bedeutet gegebenenfalls durch Halogen oder niederes Alkyl substituiertes Phenyl. Der weiter unten verwendete Ausdruck "Halogenalkyl" bezeichnet durch Halogen substituierte Alkylreste, wie Trifluormethyl. Der Ausdruck Halogen bedeutet Fluor, Chlor, Brom oder Jod.

In einer speziellen Ausführungsform umfasst die vorliegende Erfindung die Herstellung von Verbindungen der Formel I, worin $R^1$ niederes Alkoxycarbonyl, $R^2$ Wasserstoff, B niederes Alkyliden und Z niederes 2-Alkenyl bedeuten.

Die nachfolgend aufgeführten Verbindungen sind Vertreter der durch die Formel I definierten Stoffklasse:

Methyl (Z)-3-[[(2S,3S)-1-allyl-2-[(R)-2,2-dimethyl-1,3-dioxolan-4-yl]-4-oxo-3-azetidinyl]amino]-2-butenoat,

(3S,4S)-1-Allyl-3-[[(Z)-2-benzoyl-1-methylvinyl]-amino]-4-[(R)-2,2-dimethyl-1,3-dioxolan-4-yl]-2-azetidinon,

Diäthyl [[[(2S,3S)-1-allyl-2-[(R)-2,2-dimethyl-1,3-dioxolan-4-yl]-4-oxo-3-azetidinyl]amino]methylen]malonat,

Methyl (Z)-3-[[(2S,3S)-1-(2,4-dimethoxybenzyl)-2-[(R)-2,2-dimethyl-1,3-dioxolan-4-yl]-4-oxo-3-azetidinyl)-amino]-2-butenoat und

(3S,4S)-3-[[(Z)-2-Benzoyl-1-methylvinyl]amino]-1-(2,4-dimethoxybenzyl)-4-[(R)-2,2-dimethyl-1,3-dioxolan-4-yl]-2-azetidinon.

Bei der erfindungsgemässen Reaktion handelt es sich um eine dem Fachmann geläufigen Cycloaddition. Man erhält dabei eine Verbindung der obigen Formel I oder den optischen Antipoden davon, worin die Substituenten in 3- und 4-Stellung des Azetidinonringes zueinander wie erwartet cis-ständig sind. In Chemical Abstracts 94, 1396522 f (1981) wird eine Cycloaddition zu 2-Azetichinonen beschreiben, welche in den Stellungen 3 und 4 durch 1-Methyl-2-Äthoxycarbonylvinylamino und gegebenenfalls substituiertes Phenyl cis-ständig substituiert sind. Überraschenderweise hat es sich jedoch gezeigt, dass die Verwendung einer optisch aktiven Verbindung der Formel III (bzw. des optischen Antipoden davon) in der obigen Cycloaddition in hoher optischer Ausbeute zwei neue optische Zentren induziert. In den erhaltenen Produkten konnte das zweite mögliche cis-Cycloadditionsprodukt, das zum tatsächlich erhaltenen Produkt diastereoisomer wäre, nicht nachgewiesen werden.

Als reaktive organische Sulfonsäurederivate kommen für den vorliegenden Zweck in erster Linie Verbindungen der allgemeinen Formel

$R^4$-$SO_2$-X IV

worin X Halogen oder die Gruppe -$OSO_2$-$R^4$ und $R^4$ Aryl, niederes Alkyl oder niederes Halogenalkyl bedeuten, in Frage. Bevorzugte Sulfonsäurederivate sind die Verbindungen der Formel IV, worin X Halogen, insbesondere Chlor, und $R^4$ gegebenenfalls durch Halogen oder niederes Alkyl substituiertes Phenyl bedeuten, wie p-Toluolsulfonsäurechlorid, p-Chlorbenzolsulfonsäurechlorid und Benzolsulfonsäurechlorid.

Als Basen verwendet man vorzugsweise tertiäre Amine, wobei insbesondere tertiäre aliphatische Amine, wie Diisopropyläthylamin und Triäthylamin besonders geeignet sind.

Die Carbonsäure der Formel II wird in Form eines Carbonsäuresalzes in die Reaktion eingebracht. Es kommen dabei in erster Linie Alkalimetallsalze und von tertiären Aminen abgeleitete Ammoniumsalze in Frage. In einer besonders bevorzugten Ausführungsform setzt man die Alkalimetallsalze, insbesondere die Kaliumsalze von Carbonsäuren der Formel II ein.

Für das erfindungsgemässe Verfahren geeignete inerte organische Lösungsmittel sind beispielsweise Äther, wie Tetrahydrofuran, Diäthyläther, t-Butylmethyläther, Dioxan, Äthylenglykoldimethyläther oder dergleichen, halogenierte Kohlenwasserstoffe, wie Methylenchlorid, Chloroform, 1,2-Dichloräthan oder dergleichen, Acetonitril, Dimethylformamid oder dergleichen. Besonders geeignete Lösungsmittel sind halogenierte Kohlenwasserstoffe, wie Methylenchlorid. Die obige Cycloaddition wird dabei in einem Temperaturbereich von etwa -30°C bis etwa 50°C durchgeführt. Vorzugsweise liegt die Reaktionstemperatur in einem Bereich von etwa 0°C bis Raumtemperatur.

Die als Ausgangsstoffe verwendeten Verbindungen der Formel III bzw. deren optische Antipoden können hergestellt werden, indem man einen Aldehyd der allgemeinen Formel

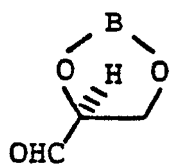

V

worin B obige Bedeutung besitzt,
bzw. dessen optischen Antipoden mit einem Amin der allgemeinen Formel

$H_2N$-Z VI

worin Z obige Bedeutung besitzt,
umsetzt. Man arbeitet dabei vorzugsweise in einem inerten organischen Lösungsmittel, beispielsweise in einem halogenierten Kohlenwasserstoff, wie Methylenchlorid, Chloroform, 1,2-Dichloräthan und dergleichen, oder in einem Kohlenwasserstoff, wie Benzol, Toluol und dergleichen. Vorzugsweise entfernt man dabei laufend das während der Reaktion entstehende Reaktionswasser, beispielsweise durch azeotrope Destillation oder durch Arbeiten in Gegenwart eines wasserentziehenden Mittels, beispielsweise in Gegenwart eines geeigneten Molekularsiebes, oder von anderen herkömmlichen Trocknungsmitteln, wie Kaliumcarbonat, Magnesiumsulfat und dergleichen. Bei azeotroper Entfernung des gebildeten Reaktionswassers arbeitet man bei der Siedetemperatur des gewählten Lösungsmittels; bei Verwendung eines wasserentziehenden Mittels arbeitet man vorzugsweise bei Raumtemperatur.

Die Verbindungen der Formel III und ihre entsprechenden optischen Antipoden, welche in der weiter oben beschriebenen Cycloaddition überraschenderweise optisch einheitliche Produkte liefern, müssen nicht notwendigerweise isoliert werden, sondern können direkt der erfindungsgemässen Cycloaddition unterworfen werden.

Die erfindungsgemäss erhältlichen Verbindungen der Formel I sind neu und können zur Herstellung von antimikrobiell wirksamen, optisch einheitlichen Stoffen verwendet werden, welche einen β-Lactamring

enthalten. Solche antimikrobiell wirksamen Stoffe können ausgehend von den erfindungsgemäss erhältlichen Verbindungen der Formel nach an sich bekannten Methoden hergestellt werden, wobei die Auswahl der Reagenzien und Reaktionsbedingungen, je nach gewünschter Zielverbindung, dem Fachmann keine Schwierigkeiten bereitet.

Die Verbindungen der Formel I können insbesondere zur Herstellung von antimikrobiell wirksamen Verbindungen der allgemeinen Formel

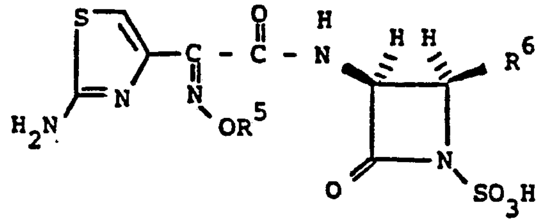

VII

worin $R^5$ Wasserstoff, niederes Alkyl oder Carboxyniederes Alkyl und $R^6$ Carbamoyl oder Carbamoyloxymethyl bedeuten,

und deren pharmazeutisch annehmbaren Salzen verwendet werden. Die Anwendung der erfindungsgemässen Cycloaddition zur Herstellung von antimikrobiell wirksamen, optisch einheitlichen, einen β-Lactamring enthaltenden Stoffen, wie den vorerwähnten Verbindungen der Formel VII, sind ebenfalls Gegenstand der vorliegenden Erfindung.

Die Verbindungen der Formel VII können beispielsweise gemäss dem nachfolgenden Reaktionsschema und den daran anschliessenden Angaben hergestellt werden. Die im Reaktionsschema verwendeten Symbole $R^1$, $R^2$, $R^3$, $R^5$, $R^6$, B und Z besitzen obige Bedeutung; Z' bedeutet eine leicht abspaltbare N-Schutzgruppe, vorzugsweise eine leicht abspaltbare Acylgruppe, wie t-Butoxycarbonyl, Trichloräthoxycarbonyl, Benzyloxycarbonyl und dergleichen.

**Reaktionsschema**

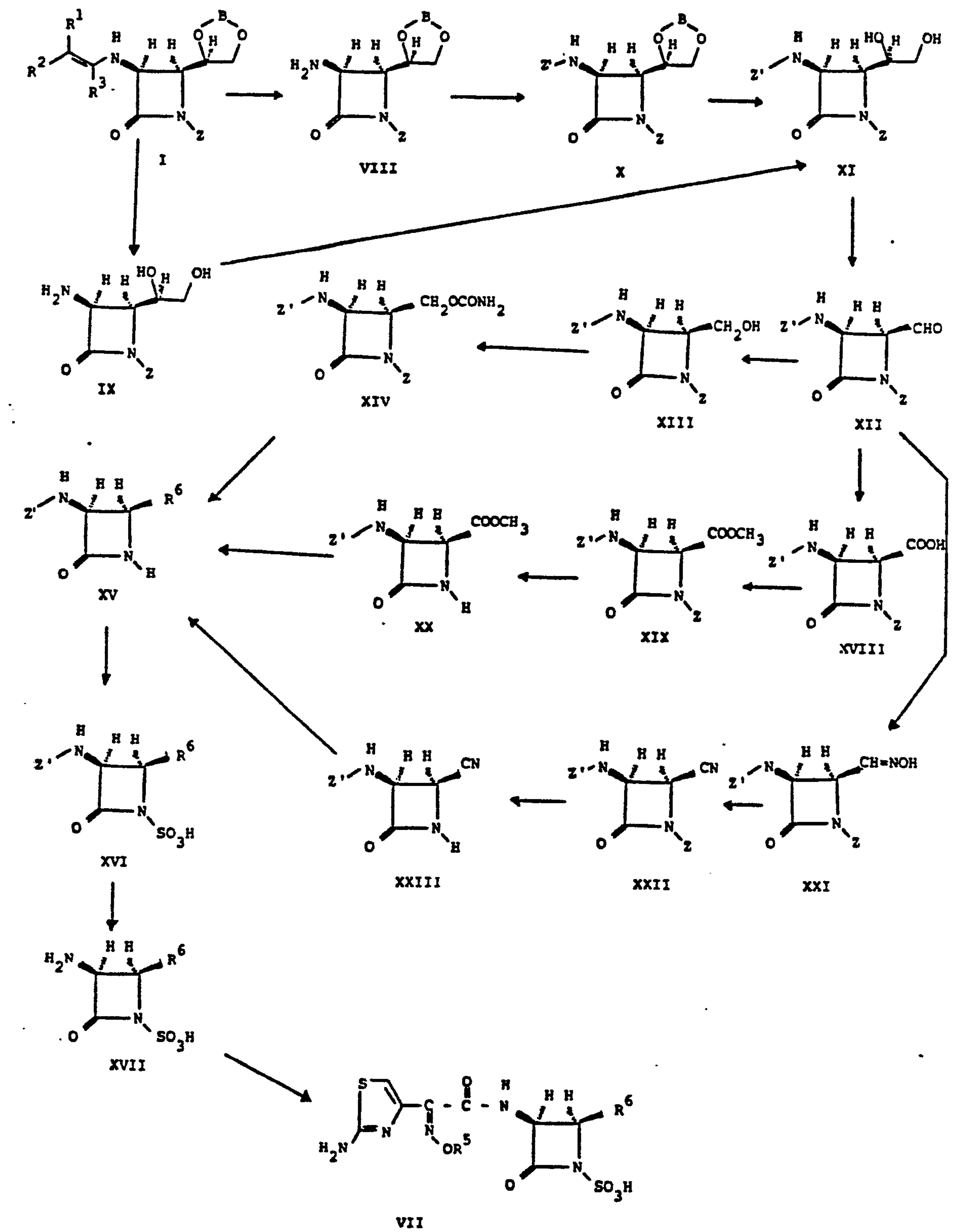

I, VIII, X, XI, IX, XIV, XIII, XII, XV, XX, XIX, XVIII, XVI, XXIII, XXII, XXI, XVII, VII

Durch milde saure Hydrolyse einer Verbindung der Formel I erhält man eine Verbindung der Formel VIII. Die Hydrolyse einer Verbindung der Formel I kann beispielsweise dadurch bewerkstelligt werden, dass man die Verbindung der Formel I in Gegenwart von Wasser und gegebenenfalls einem mit Wasser mischbaren Lösungsmittel, wie Aceton, Tetrahydrofuran, Dioxan, Dimethylsulfoxid, Dimethylformamid oder dergleichen, mit einer Säure behandelt. Als Säuren kommen beispielsweise Mineralsäuren, wie Salzsäure und Schwefelsäure, oder organische Säuren, wie p-Toluolsulfonsäure, Pyridinium-p-toluolsulfonat oder dergleichen, oder schwefelsaure Ionenaustauscher in Frage. Je nach angewendeten Bedingungen wird bei

dieser Reaktion auch der Dioxolanring gespalten, wobei man eine Verbindung der Formel IX erhält.

Durch Behandeln einer Verbindung der Formel VIII oder IX mit einem die Gruppe Z' liefernden Mittel erhält man eine Verbindung der Formel X bzw. XI. Geeignete, die Gruppe Z' liefernde Mittel sind beispielsweise Chlorameisensäureester, wie Chlorameisensäurebenzylester, Chlorameisensäure-t-butylester, Chlorameisensäure-2,2,2-trichloräthylester und dergleichen. Diese Reaktion erfolgt zweckmässigerweise in einem inerten organischen Lösungsmittel, beispielsweise in einem halogenierten Kohlenwasserstoff, wie Methylenchlorid, Chloroform und dergleichen, und zweckmässigerweise in Gegenwart eines säurebindenden Mittels, wie Butylenoxid, Triäthylamin, Chinuclidin, usw. Zweckmässigerweise arbeitet man bei Raumtemperatur.

Die Hydrolyse einer Verbindung der Formel X zu einer Verbindung der Formel XI erfolgt vorzugsweise unter milden sauren Bedingungen. In einer bevorzugten Ausführungsform bewerkstelligt man die gewünschte Reaktion durch Umacetalisieren in einem niederen Alkohol, wie Methanol oder Äthanol, und in Gegenwart eines geeigneten sauren Katalysators. Geeignete Katalysatoren sind beispielsweise schwefelsaure Ionenaustauscher, Pyridinium-p-toluolsulfonat, p-Toluolsulfonsäure und dergleichen. Man arbeitet dabei vorzugsweise bei Raumtemperatur. Die Hydrolyse kann jedoch ohne weiteres auch in Gegenwart von Wasser und einem mit Wasser mischbaren Lösungsmittel, wie Tetrahydrofuran, Dioxan, Dimethylsulfoxid, Dimethylformamid oder dergleichen, durchgeführt werden.

Die Spaltung der Diolgruppierung in einer Verbindung der Formel XI erfolgt nach an sich bekannten und jedem Fachmann geläufigen Methoden, und kann beispielsweise mit Natriumperjodat in Wasser bewerkstelligt werden. Gegebenenfalls kann man diese Reaktion in Gegenwart eines Lösungsvermittlers, wie Tetrahydrofuran, Dioxan, Methanol, Äthanol oder dergleichen, durchführen. Man erhält bei dieser Reaktion einen Aldehyd der Formel XII.

Die Reduktion eines Aldehyds der Formel XII zu einem primären Alkohol der Formel XIII erfolgt ebenfalls nach an sich bekannten und jedem Fachmann geläufigen Methoden, beispielsweise durch Behandeln mit Natriumborhydrid in einem niederen Alkohol, wie Äthanol, Isopropanol oder dergleichen.

Durch Umsetzen einer Verbindung der Formel XIII mit Chlorsulfonylisocyanat in einem inerten organischen Lösungsmittel erhält man eine Verbindung der Formel XIV. Geeignete Lösungsmittel sind z. B. Äther, wie Diäthyläther, t-Butylmethyläther und Äthylenglykoldimethyläther, halogenierte Kohlenwasserstoffe, wie Methylenchlorid und Chloroform, Acetonitril, Dimethylformamid, Dimethylsulfoxid, Aceton und dergleichen. Die Reaktion wird vorzugsweise in einem Temperaturbereich von etwa 0°C bis etwa Raumtemperatur durchgeführt.

Durch Abspalten der mit Z bezeichneten Schutzgruppe aus einer Verbindung der Formel XIV erhält man eine entsprechende Verbindung der Formel XV. Die Abspaltung einer 2,4-Di(niederen Alkoxy)benzylgruppe erfolgt zweckmässigerweise durch milde Oxidation. Geeignete Oxidationsmittel sind beispielsweise Kaliumperoxidisulfat oder Ammoniumperoxidisulfat, wobei man in Wasser bei einem pH von 6-8 arbeitet. Als Puffer eignet sich beispielsweise Dinatriumhydrogenphosphat.

Eine niedere 2-Alkenylgruppe wird dadurch abgespalten, dass man diese zu einer 1-Alkenylgruppe isomerisiert und letztere anschliessend oxidativ abspaltet. Die Isomerisierung der Doppelbindung erfolgt vorzugsweise mit Hilfe eines Isomerisierungskatalysators, z. B. mit einem Palladiumdihalogenid, wie Palladiumdichlorid oder mit einem Tris(triphenylphosphin)rhodium(I)halogenid, z. B. mit dem entsprechenden Chlorid, oder auch mit Palladium auf Kohle in Gegenwart einer Protonensäure, wie Salzsäure oder Phosphorsäure. Als Lösungsmittel verwendet man zweckmässigerweise Äthanol, Methylenchlorid oder Mischungen davon mit Wasser. Die Reaktionstemperatur liegt zweckmässigerweise in einem Bereich von etwa 50° C bis zum Siedepunkt des Reaktionsgemisches.

Die oxidative Abspaltung der niederen 1-Alkenylgruppe kann beispielsweise durch Behandeln mit einem Alkalimetallpermanganat, z. B. Kaliumpermanganat, bewerkstelligt werden, wobei man vorzugsweise eine wässrige Kaliumpermanganatlösung verwendet. Wahlweise kann man die Oxidation mit Hilfe eines Alkalimetallperjodates, z. B. mit Kaliumperjodat, in Gegenwart einer katalytischen Menge des erwähnten Alkalimetallpermanganates durchführen. Die Reaktion erfolgt vorzugsweise in einem wässrigen, gepufferten, insbesondere auf pH 7-8 gepufferten Medium, kann aber auch in einem mit Wasser mischbaren organischen Lösungsmittel, z. B. in Aceton, Dimethoxyäthan, Dioxan oder Tetrahydrofuran, unter Zusatz einer schwachen organischen Base, wie Pyridin, oder in einer Mischung eines dieser Lösungsmittel mit dem erwähnten wässrigen Puffer, durchgeführt werden. Man kann die Reaktion jedoch auch in einem Zweiphasensystem unter Verwendung eines Phasentransferkatalysators durchführen.

Als organische mit Wasser nicht mischbare Phase kann man beispielsweise Methylenchlorid oder Benzol verwenden. Als Phasentransferkatalysatoren können die üblicherweise hierfür verwendeten eingesetzt werden, insbesondere organische quaternäre Ammoniumhalogenide, wie Benzyltriäthylammoniumchlorid, Tetra-n-Butylammoniumbromid und Cetyltrimethylammoniumbromid. Die oxidative Abspaltung wird vorzugsweise bei einer Temperatur zwischen etwa 0° C und 25° C durchgeführt.

Die Verbindungen der Formel XV, worin $R^6$ Carbamoyl bedeutet, können erhalten werden, indem man einen Aldehyd der Formel XII nach an sich bekannten Methoden zu einer Carbonsäure der Formel XVIII oxidiert, die Carbonsäure der Formel XVIII beispielsweise mit Methyljodid in Gegenwart von Kaliumcarbonat verestert, aus der erhaltenen Verbindung der Formel XIX die mit Z bezeichnete Schutzgruppe wie oben beschrieben abspaltet und die erhaltene Verbindung der Formel XX mit Hydroxylamin behandelt.

Die Verbindungen der Formel XV, worin $R^6$ Carbamoyl bedeutet, können jedoch auch erhalten werden,

indem man eine Verbindung der Formel XII mit Hydroxylamin behandelt, das erhaltene Oxim der Formel XXI in an sich bekannter Weise in das Nitril der Formel XXII überführt, daraus wie oben näher beschrieben die mit Z bezeichnete Schutzgruppe abspaltet und in der erhaltenen Verbindung der Formel XXIII die Nitrilgruppe in an sich bekannter Weise zur Carbamoylgruppe verseift.

Durch Behandeln einer Verbindung der Formel XV mit Schwefeltrioxid oder einem geeigneten Komplex des Schwefeltrioxids erhält man eine Verbindung der Formel XVI. Geeignete Schwefeltrioxidkomplexe sind beispielsweise Komplexe mit Pyridin, Trimethylamin, Picolin, Dimethylformamid und dergleichen. Als Lösungsmittel verwendet man zweckmässigerweise einen Äther, wie Dioxan, Pyridin, Acetonitril, Dimethylformamid oder dergleichen. Acetonitril ist das bevorzugte Lösungsmittel. Die Reaktion wird vorzugsweise zwischen etwa 0° C bis 80° C durchgeführt.

Durch Abspaltung der mit Z' bezeichneten Schutzgruppe aus einer Verbindung der Formel XVI erhält man eine Verbindung der Formel XVII. Eine Benzyloxycarbonylgruppe kann beispielsweise hydrogenolytisch abgespalten werden, beispielsweise durch Behandeln mit elementarem Wasserstoff in Gegenwart von Palladium auf Kohle. Eine t-Butoxycarbonylgruppe kann beispielsweise durch Behandeln mit Trifluoressigsäure oder Ameisensäure abgespalten werden. Eine Trichloräthoxycarbonylgruppe lässt sich beispielsweise durch Behandeln mit Zink in Gegenwart einer Säure, wie Essigsäure oder Salzsäure, abspalten.

Durch Acylierung einer Verbindung der Formel XVII mit einem reaktionsfähigen funktionellen Derivat einer Carbonsäure der allgemeinen Formel

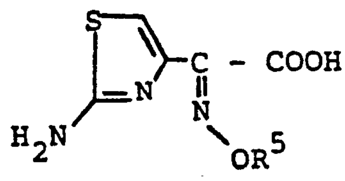

XXIV

worin $R^5$ obige Bedeutung besitzt, erhält man schliesslich die gewünschte Zielverbindung der Formel VII. Es sei noch erwähnt, dass die Verbindung der Formel XXIV geeignet geschützt werden muss, wenn $R^5$ Wasserstoff oder niederes Carboxyalkenyl bedeutet; nach erfolgter Acylierung wird dann die Schutzgruppe wieder entfernt. Als reaktionsfähige funktionelle Derivate von Verbindungen der Formel XXIV kann man beispielsweise entsprechende Säureanhydride, gemischte Säureanhydride, Benzthiazolylthioester und dergleichen verwenden. Die nachfolgenden Beispiele dienen der näheren Erläuterung der vorliegenden Erfindung; sie sollen dieselbe jedoch in keiner Weise einschränken. Alle Temperaturen sind in Celsius Graden angegeben.

**Beispiel 1**

a) Man löst 12 g (92,16 mMol) Isopropyliden-L-glyceraldehyd in 200 ml Methylenchlorid, versetzt zuerst mit 60 g Magnesiumsulfat und dann tropfenweise über einen Zeitraum von 10 Minuten mit 5,26 g (92,16 mMol) 3-Amino-1-propen in 50 ml Methylenchlorid und rührt die Suspension während 5 Stunden bei Raumtemperatur. Man filtriert das Magnesiumsulfat ab und dampft die klare farblose Lösung am Rotationsverdampfer ein. Man erhält 14,6 g (86,3 mMol; 93,6 %) reines Isopropyliden-L-glyceraldehyd-allyl-imin.

b) Man löst 8,64 g (51,1 mMol) Isopropyliden-L-glyceraldehyd-allyl-imin in 400 ml Methylenchlorid, versetzt zuerst mit 10,32 g (102,2 mMol) Triäthylamin und dann mit 10,79 g (51,1 mMol) N-(1-Methyl-2-methoxycarbonylvinyl)aminoessigsäure-Kaliumsalz, kühlt die Suspension auf 0° C ab und versetzt tropfenweise innerhalb von 5 Minuten mit 9,74 g (51,1 mMol) p-Toluolsulfonsäurechlorid in 50 ml Methylenchlorid. Man entfernt das Kühlbad, rührt das Reaktionsgemisch während 8 Stunden bei Raumtemperatur, versetzt mit 200 ml Wasser trennt die organische Phase ab und dampft sie ein. Das Rohprodukt wird an Kieselgel unter Eluieren mit Hexan/Essigester (8 : 2) chromatographiert. Man erhält 13,0 g (40,0 mMol; 78 %) Methyl (Z)-3-[[(2S,3S)-1-allyl-2-[(R)-2,2-dimethyl-1,3-dioxolan-4-yl]-4-oxo-3-azetidinyl]amino]-2-butenoat als Öl, das beim Stehenlassen langsam erstarrt. Durch Kristallisieren aus Äther/Hexan erhält man ein Produkt vom Schmelzpunkt 98°.

**Beispiel 2**

Man löst 1,7 g (10 mMol) Isopropyliden-L-glyceraldehydallyl-imin in 80 ml Methylenchlorid, versetzt zuerst mit 3,03 g (30 mMol) Triäthylamin und dann mit 2,6 g (10 mMol) N-(1-Methyl-2-benzoylvinyl)aminoessigsäure-Kaliumsalz und kühlt die Suspension auf 0° ab. Man tropft 2,85 g (15 mMol) p-Toluolsulfonsäurechlorid in 10 ml Methylenchlorid dazu, rührt während 5 Stunden bei Raumtemperatur, wäscht das Gemisch mit 100 ml Wasser und dampft ein. Das Rohprodukt wird an Kieselgel chromatographiert, wobei man mit Hexan/Essigester (7 : 3) eluiert. Man erhält 1,5 g (4,04 mMol; 40 %) (3S,4S)-1-Allyl-3-[[(Z)-2-benzoyl-1-methylvinyl]amino]-4-[(R)-2 2-dimethyl-1,3-dioxolan-4-yl]-2-azetidinon als reines Öl, das aus einem Gemisch Äther/Hexan auskristallisiert und

dann einen Schmelzpunkt von 154-156° hat.

## Beispiel 3

Man löst 1,7 g (10 mMol) Isopropyliden-L-glyceraldehydallyl-imin in 80 ml Methylenchlorid und versetzt zuerst mit 3,03 g (30 mMol) Triäthylamin und dann mit 2,8 g (10 mMol) N-(2,2-Diäthoxycarbonylvinyl)aminoessigsäure-Kaliumsalz. Man tropft dann zur eisgekühlten Lösung 2,85 g (15 mMol) p-Toluolsulfonsäurechlorid in 20 ml Methylenchlorid und rührt das Gemisch während 5 Stunden bei Raumtemperatur. Anschliessend wäscht man das Gemisch mit 100 ml Wasser, dampft die organische Phase ein und chromatographiert das erhaltene Rohprodukt an Kieselgel unter Eluieren mit Hexan/Essigester (7 : 3). Man erhält 1,15 g (2,9 mMol; 29 %) reines Diäthyl [[[(2S,3S)-1-allyl-2-[(R)-2,2-dimethyl-1,3-dioxolan-4-yl]-4-oxo-3-azetidinyl]amino]methylen]malonat als Öl.

IR-Spektrum (Film): u.a. Banden bei 1755, 1740, 1700, 1660 und 1600 $cm^{-1}$.

## Beispiel 4

Man löst 3,75 g (13,44 mMol) Isopropyliden-L-glyceraldehyd-(2,4-dimethoxybenzyl)imin (erhalten gemäss Angaben von Beispiel 1a) aus Isopropyliden-L-glyceraldehyd und 2,4-Dimethoxybenzylamin) in 150 ml Methylenchlorid und versetzt mit 3,25 g (32,25 mMol) Triäthylamin. 3,40 g (16,12 mMol) N-(1-Methyl-2-methoxycarbonylvinyl)aminoessigsäure-Kaliumsalz werden zugegeben und die Suspension wird auf 0° abgekühlt. Man tropft innerhalb von 5 Minuten 3,40 g (16,12 mMol) p-Toluolsulfonsäurechlorid in 50 ml Methylenchlorid dazu und rührt das Gemisch während 15 Stunden bei Raumtemperatur. Nach Waschen mit 100 ml Wasser und Eindampfen der organischen Phase wird der Rückstand durch Chromatographieren an Kieselgel mit Hexan/Essigester (7 : 3) als Elutionsmittel gereinigt, und man erhält 3,45 g (7,94 mMol; 59 %) reines Methyl (Z)-3-[[(2S,3S)-1-(2,4-dimethoxybenzyl)-2-[(R)-2,2-dimethyl-1,3-dioxolan-4-yl]-4-oxo-3-azetidinyl]amino]-2-butenoat als Öl, das langsam erstarrt. Nach Kristallisieren aus Äther/Hexan hat das Produkt einen Schmelzpunkt von 121°.

## Beispiel 5

Man löst 2,8 g (10 mMol) Isopropyliden-L-glyceraldehyd-(2,4-dimethoxybenzyl)imin in 125 ml Methylenchlorid und versetzt mit 3,03 g (30 mMol) Triäthylamin. 2,6 g (10 mMol) N-(1-Methyl-1-benzoyl-vinyl)aminoessigsäure-Kaliumsalz werden dann zugegeben, und die Suspension wird bei 0° stark gerührt. Man tropft langsam 2,85 g (15 mMol) p-Toluolsulfonsäurechlorid in 50 ml Methylenchlorid dazu und rührt das Gemisch während 5 Stunden bei Raumtemperatur. Man versetzt dann mit 100 ml Wasser, trennt die organische Phase ab und dampft ein. Den Rückstand chromatographiert man an Kieselgel mit Hexan/Essigester (7 : 3) als Elutionsmittel. Man erhält 1,4 g (2,91 mMol; 29 %) reines (3S,4S)3-[[(Z)-2-Benzoyl-1-methylvinyl]amino]-1-(2,4-dimethoxybenzyl)-4-[(R)-2 2-dimethyl-1,3-dioxolan-4-yl]-2-azetidinon, das aus Äther/Hexan auskristallisiert und dann einen Schmelzpunkt von 177-179° hat.

## Beispiel 6

Man löst 6,48 g (20 mMol) Methyl (Z)-3-[[(2S,3S)-1-allyl-2-[(R)-2,2-dimethyl-1,3-dioxolan-4-yl]-4-oxo-3-azetidinyl]amino]-2-butenoat in 40 ml Aceton und versetzt mit 3,80 g (20 mMol) p-Toluolsulfonsäuremonohydrat in 20 ml Aceton. Die klare Lösung wird während 15 Minuten bei Raumtemperatur gerührt und dann langsam mit 120 ml Äther versetzt. Das ausgefallene Produkt wird abfiltriert und getrocknet. Man erhält 6,4 g (15,3 mMol; 77 %) reines (3S,4S)-cis-3-Amino-1-allyl-4-[(R)-2,2-dimethyl-1,3-dioxolan-4-yl]-2-azetidinon-p-Toluolsulfonsäuresalz vom Schmelzpunkt 165°.

Identische Produkte erhält man, wenn man (3S,4S)-1-Allyl-3-[[(Z)-2-benzoyl-1-methylvinyl]amino]-4-[(R)-2,2-dimethyl-1,3-dioxolan-4-yl]-2-azetidinon und Diäthyl [[[(2S,3S)-1-allyl-2-[(R)-2,2-dimethyl-1,3-dioxolan-4-yl]-4-oxo-3-azetidinyl]amino]methylen]malonat den gleichen Reaktionsbedingungen unterwirft. Die Ausbeuten liegen zwischen 75 und 80 %.

**Patentansprüche**

1. Verfahren zur Herstellung von optisch einheitlichen β-Lactamen der allgemeinen Formel

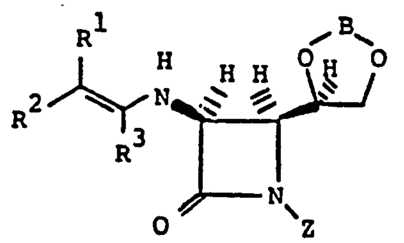

I

worin $R^1$ niederes Alkanoyl, niederes Alkoxycarbonyl oder Benzoyl, $R^2$ Wasserstoff, niederes Alkyl, niederes Alkanoyl, niederes Alkoxycarbonyl oder Cyano, $R^3$ Wasserstoff oder niederes Alkyl, B niederes Alkyliden, niederes Cycloalkyliden oder Carbonyl und Z niederes 2-Alkenyl oder 2,4-Di(niederes Alkoxy)benzyl bedeuten, wobei die mit nieder bezeichneten Reste höchstens 7 Kohlenstoffatome besitzen,

und ihren entsprechenden optischen Antipoden, dadurch gekennzeichnet, dass man ein Salz einer Carbonsäure der allgemeinen Formel

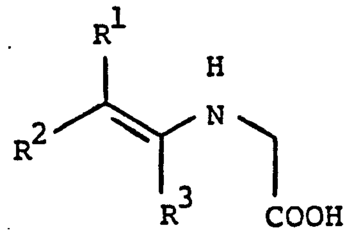

II

worin $R^1$, $R^2$ und $R^3$ obige Bedeutung besitzen, in Gegenwart eines reaktiven Derivates einer organischen Sulfonsäure und einer Base mit einer optisch einheitlichen Verbindung der allgemeinen Formel

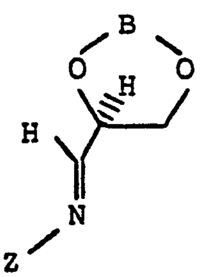

III

worin B und Z obige Bedeutung besitzen, oder dem optischen Antipoden davon umsetzt.

2. Verfahren gemäss Anspruch 1, dadurch gekennzeichnet, dass $R^1$ niederes Alkoxycarbonyl und $R^2$ Wasserstoff bedeuten.

3. Verfahren gemäss Anspruch 1 oder 2, dadurch gekennzeichnet, dass B niederes Alkyliden bedeutet.

4. Verfahren gemäss einem der Ansprüche 1 bis 3, dadurch gekennzeichnet, dass Z 2-Alkenyl bedeutet.

5. Verfahren gemäss Anspruch 1, dadurch gekennzeichnet, dass man Methyl (Z)-3-[[(2S,3S)-1-allyl-2-[(R)-2,2-dimethyl-1,3-dioxolan-4-yl]-4-oxo-3-azetidinyl]amino]-2-butenoat herstellt.

6. Verfahren gemäss Anspruch 1, dadurch gekennzeichnet, dass man (3S,4S)-1-Allyl-3-[[(Z)-2-benzoyl-1-methylvinyl]-amino]-4-[(R)-2,2-dimethyl-1,3-dioxolan-4-yl]-2-azetidinon herstellt.

7. Verfahren gemäss Anspruch 1, dadurch gekennzeichnet, dass man Diäthyl [[[(2S,3S)-1-allyl-2-[(R)-2,2-dimethyl-1,3-dioxolan-4-yl]-4-oxo-3-azetidinyl]amino]methylen]-malonat herstellt.

8. Verfahren gemäss Anspruch 1, dadurch gekennzeichnet, dass man Methyl (Z)-3-[[(2S,3S)-1-(2,4-dimethoxybenzyl)-2-[(R)-2,2-dimethyl-1,3-dioxolan-4-yl]-4-oxo-3-azetidinyl]-amino]-2-butenoat herstellt.

9. Verfahren gemäss Anspruch 1, dadurch gekennzeichnet, dass man (3S,4S)-3-[[(Z)-2-Benzoyl-1-methylvinyl]amino]-1-(2,4-dimethoxybenzyl)-4-[(R)-2,2-dimethyl-1,3-dioxolan-4-yl]-2-azetidinon herstellt.

10. Verfahren gemäss einem der Ansprüche 1 bis 9, dadurch gekennzeichnet, dass das reaktive Derivat einer organischen Sulfonsäure eine Verbindung der allgemeinen Formel

$R^4$-$SO_2$-X IV

worin X Halogen oder die Gruppe -$OSO_2$-$R^4$ und $R^4$ Aryl, niederes Alkyl oder niederes Halogenalkyl bedeuten, ist, wobei die mit nieder bezeichneten Reste höchstens 7 Kohlenstoffatome besitzen.

11. Verfahren gemäss Anspruch 10, dadurch gekennzeichnet, dass X Halogen, insbesondere Chlor, und $R^4$ gegebenenfalls durch Halogen oder niederes Alkyl substituiertes Phenyl bedeuten.

12. Verfahren gemäss einem der Ansprüche 1 bis 11, dadurch gekennzeichnet, dass die Base ein tertiäres Amin ist.

13. Verfahren gemäss einem der Ansprüche 1 bis 12, dadurch gekennzeichnet, dass man ein Alkalimetallsalz, insbesondere das Kaliumsalz einer Carbonsäure der Formel II verwendet.

14. Anwendung eines Verfahrens gemäss einem der Ansprüche 1 bis 13 bei der Herstellung von antimikrobiell wirksamen, optisch einheitlichen, einen β-Lactamring enthaltenden Stoffen.

15. Anwendung eines Verfahrens gemäss einem der Ansprüche 1 bis 13 bei der Herstellung von antimikrobiell wirksamen Stoffen der allgemeinen Formel

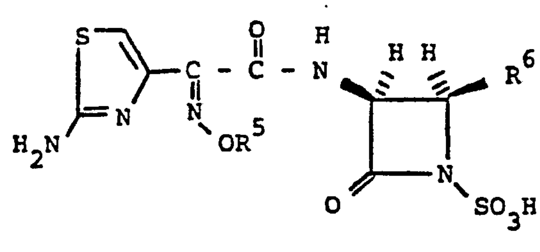

VII

worin $R^5$ Wasserstoff, niederes Alkyl oder Carboxy- niederes Alkyl und $R^6$ Carbamoyl oder Carbamoyloxymethyl bedeuten, und deren pharmazeutisch annehmbaren Salzen.

## Claims

1. A process for the manufacture of optically uniform β-lactams of the general formula

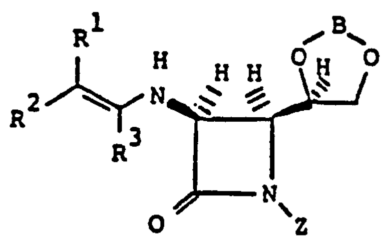

I

wherein $R^1$ signifies lower alkanoyl, lower alkoxycarbonyl or benzoyl, $R^2$ signifies hydrogen, lower alkyl, lower alkanoyl, lower alkoxycarbonyl or cyano, $R^3$ signifies hydrogen or lower alkyl, B signifies lower alkylidene, lower cycloalkylidene or carbonyl and Z signifies lower 2-alkenyl or 2,4-di(lower alkoxy)benzyl, whereby the residues denoted as lower have a maximum of 7 carbon atoms,

and their corresponding optical antipodes, characterized by reacting a salt of a carboxylic acid of the general formula

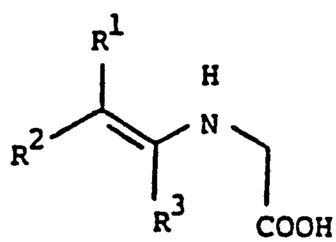

II

wherein $R^1$, $R^2$ and $R^3$ have the above significance,

in the presence of a reactive derivative of an organic sulphonic acid and a base with an optically uniform compound of the general formula

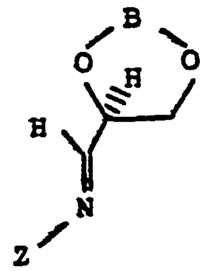

III

wherein B and Z have the above significance, or the optical antipode thereof.

2. A process in accordance with claim 1, characterized in that $R^1$ signifies lower alkoxycarbonyl and $R^2$ signifies hydrogen.

3. A process in accordance with claim 1 or 2, characterized in that B signifies lower alkylidene.

4. A process in accordance with any one of claims 1 to 3, characterized in that Z signifies 2-alkenyl.

5. A process in accordance with claim 1, characterized in that methyl (Z)-3-[[(2S,3S)-1-allyl-2-[(R)-2,2-dimethyl-1,3-dioxolan-4-yl]-4-oxo-3-azetidinyl]amino]-2-butenoate is manufactured.

6. A process in accordance with claim 1, characterized in that (3S,4S)-1-allyl-3-[[(Z)-2-benzoyl-1-methylvinyl]amino]-4-[(R)-2,2-dimethyl-1,3-dioxolan-4-yl]-2-azetidinone is manufactured.

7. A process in accordance with claim 1, characterized in that diethyl [[[(2S,3S)-1-allyl-2-[(R)-2,2-dimethyl-1,3-dioxolan-4-yl]-4-oxo-3-azetidinyl]amino]-methylene]malonate is manufactured.

8. A process in accordance with claim 1, characterized in that methyl (Z)-3-[[(2S,3S)-1-(2,4-dimethoxybenzyl)-2-[(R)-2,2-dimethyl-1,3-dioxolan-4-yl]-4-oxo-3-azetidinyl]amino]-2-butenoate is manufactured.

9. A process in accordance with claim 1, characterized in that (3S,4S)-3-[[(Z)-2-benzoyl-1-methylvinyl]-amino]-1-(2,4-dimethoxybenzyl)-4-[(R)-2,2-dimethyl-1,3-dioxolan-4-yl]-2-azetidinone is manufactured.

10. A process in accordance with any one of claims 1 to 9, characterized in that the reactive derivative of an organic sulphonic acid is a compound of the general formula

$R^4$-$SO_2$-X IV

wherein X signifies Halogen or the group -$OSO_2$-$R^4$ and $R^4$ signifies aryl, lower alkyl or lower haloalkyl, whereby the residues denoted as lower have a maximum of 7 carbon atoms.

11. A process in accordance with claim 10, characterized in that X signifies halogen, especially chlorine, and $R^4$ signifies phenyl optionally substituted by halogen or lower alkyl.

12. A process in accordance with any one of claims 1 to 11, characterized in that the base is a tertiary amine.

13. A process in accordance with any one of claims 1 to 12, characterized in that an alkali metal salt, especially the potassium salt, of a carboxylic acid of formula II is used.

14. The use of a process in accordance with any one of claims 1 to 13 in the manufacture of antimicrobially active, optically uniform substances containing a β-lactam ring.

15. The use of a process in accordance with any one of claims 1 to 13 in the manufacture of antimicrobially active substances of the general formula

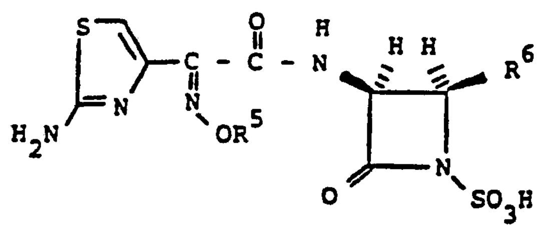

VII

wherein $R^5$ signifies hydrogen, lower alkyl or carboxy-lower alkyl and $R^6$ signifies carbamoyl or carbamoyloxymethyl,
and their pharmaceutically acceptable salts.

**Revendications**

1. Procédé de préparation de β-lactames optiquement unitaires de formule générale

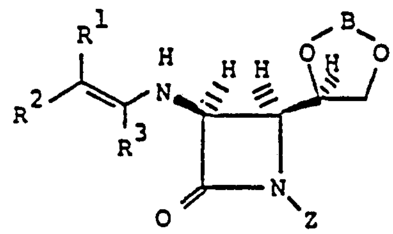

I

où $R^1$ représente un alcanoyle inférieur, alcoxycarbonyle inférieur ou benzoyle, $R^2$ représente un hydrogéne, alcoyle inférieur, alcanoyle inférieur, alcoxycarbonyle inférieur ou cyano, $R^3$ représente un hydrogène ou un alcoyle inférieur, B représente un alcoylidène inférieur, cycloalcoylidène inférieur ou carbonyle et Z représente un 2-alcényle inférieur ou un 2,4-di(alcoxy inférieur)benzyle, où les radicaux décrits comme inférieurs possédent au plus 7 atomes de carbone,

et de leurs antipodes optiques correspondants, caractérisé en ce qu'on fait réagir un sel d'un acide carboxylique de formule générale

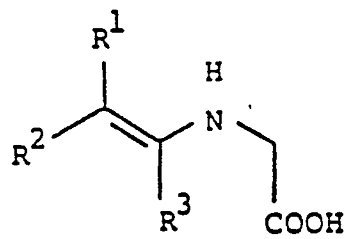

II

où $R^2$ et $R^3$ ont la signification donnée ci-dessus,

en présence d'un dérivé réactif d'un acide sulfonique organique et d'une base avec un composé optiquement unitaire de formule générale

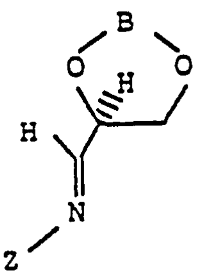

III

où B et Z ont la signification donnée ci-dessus, ou son antipode optique.

2. Procédé selon la revendication 1, caractérisé en ce que $R^1$ représente un alcoxycarbonyle inférieur et $R^2$ un hydrogène.

3. Procédé selon la revendication 1 ou 2, caractérisé en ce que B représente un alcoylidzène inférieur.

4. Procédé selon l'une des revendications 1 à 3, caractérisé en ce que Z représente un 2-alcényle.

5. Procédé selon la revendication 1, caractérisé en ce qu'on prépare le méthyl (Z)-3-[[(2S,3S)-1-allyl-2-[(R)-2,2-diméthyl-1,3-dioxolan-4-yl]-4-oxo-3-azétidinyl]amino]-2-buténoate.

6. Procédé selon la revendication 1, caractérisé en ce qu'on prépare la (3S,4S)-1-allyl-3-[[(Z)-2-benzoyl-1-méthylvinyl]-amino]-4-[(R)-2,2-diméthyl-1,3-dioxolan-4-yl]-2-azétidinone.

7. Procédé selon la revendication 1, caractérisé en ce qu'on prépare le diéthyl [[[(2S,3S)-1-allyl-2-[(R)-2,2-diméthyl-1,3-dioxolan-4-yl]-4-oxo-3-azétidinyl]amino]méthylène]malonate.

8. Procédé selon la revendication 1, caractérisé en ce qu'on prépare le méthyl (Z)-3-[[(2S,3S)-1-(2,4-diméthoxybenzyl)-2-[(R)-2,2-diméthyl-1,3-dioxolan-4-yl]-4-oxo-3-azétidinyl]-amino]-2-buténoate.

9. Procédé selon la revendication 1, caractérisé en ce qu'on prépare la (3S,4S)-3-[[(Z)-2-benzoyl-1-méthylvinyl]amino]-1-(2,4-diméthoxybenzyl)-4-[(R)-2,2-diméthyl-1,3-dioxolan-4-yl]-2-azétidinone.

10. Procédé selon l'une des revendications 1 à 9, caractérisé en ce que le dérivé réactif d'un acide sulfonique organique est un composé de formule générale

$R^4$-$SO_2$-X IV

où X représente un halogène ou le groupe $OSO_2$ -$R^4$ et $R^4$ un aryle, alcoyle inférieur ou halogénalcoyle inférieur, où les radicaux décrits comme inférieurs possédent au plus 7 atomes de carbone.

11. Procédé selon la revendication 10, caractérisé en ce que X représente un halogène, en particulier un

chlore, et $R^4$ un phényle éventuellement substitué par un halogène ou un alcoyle inférieur.

12. Procédé selon l'une des revendications 1 à 11, caractérisé en ce que la base est une amine tertiaire.

13. Procédé selon l'une des revendications 1 à 12, caractérisé en ce qu'on utilise un sel de métal alcalin, en particulier le sel de potassium d'un acide carboxylique de formule II.

14. Application d'un procédé selon l'une des revendications 1 à 13 à la préparation de produits à action antimicrobienne, optiquement unitaires, contenant un noyau β-lactame.

15. Application d'un procédé selon l'une des revendications 1 à 13 à la préparation de produits à action antimicrobienne de formule générale

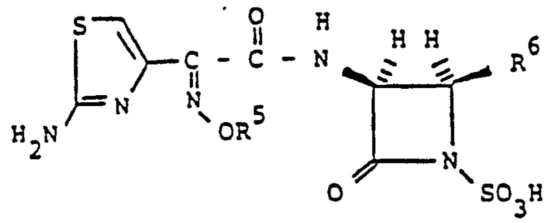

VII

où $R^5$ représente un hydrogène, un alcoyle inférieur ou un carboxy-alcoyle inferieur et $R^6$ représente un carbamoyle ou un carbamoyloxyméthyle, et de leurs sels pharmaceutiquement acceptables.